(19)

Europäisches
Patentamt
European
Patent Office
Office européen
des brevets

(11) **EP 3 220 811 B1**

(12) **EUROPEAN PATENT SPECIFICATION**

(45) Date of publication and mention
of the grant of the patent:
**18.10.2023 Bulletin 2023/42**

(21) Application number: **15860209.4**

(22) Date of filing: **17.11.2015**

(51) International Patent Classification (IPC):
*A61B 5/021* (2006.01)    *G09B 23/30* (2006.01)
*A61B 5/02* (2006.01)    *A61B 5/0285* (2006.01)
*G09B 23/28* (2006.01)    *A61B 5/0295* (2006.01)

(52) Cooperative Patent Classification (CPC):
**A61B 5/0285; A61B 5/02007; A61B 5/02125;
G09B 23/28; G09B 23/288; G09B 23/30;**
A61B 5/0295; A61B 5/352

(86) International application number:
**PCT/US2015/061190**

(87) International publication number:
**WO 2016/081519 (26.05.2016 Gazette 2016/21)**

(54) **BLOOD PRESSURE AND ARTERIAL COMPLIANCE ESTIMATION FROM ARTERIAL SEGMENTS**

BLUTDRUCK- UND ARTERIENDEHNBARKEITSSCHÄTZUNG AUSGEHEND VON
ARTERIENSEGMENTEN

ESTIMATION DE LA PRESSION ARTÉRIELLE ET DE LA COMPLIANCE ARTÉRIELLE À PARTIR
DE SEGMENTS ARTÉRIELS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.11.2014 US 201462080740 P
17.11.2014 US 201462080738 P**

(43) Date of publication of application:
**27.09.2017 Bulletin 2017/39**

(73) Proprietors:
• **Borkholder, David A.**
**Canandaigua, NY 14424 (US)**
• **Liberson, Alexander S.**
**Pittsford, NY 14534 (US)**
• **Lillie, Jeffrey S.**
**Mendon, NY 14506 (US)**

(72) Inventors:
• **Borkholder, David A.**
**Canandaigua, NY 14424 (US)**
• **Liberson, Alexander S.**
**Pittsford, NY 14534 (US)**
• **Lillie, Jeffrey S.**
**Mendon, NY 14506 (US)**

(74) Representative: **Adamson Jones
Park View House
58 The Ropewalk
Nottingham NG1 5DW (GB)**

(56) References cited:
**WO-A1-2014/077233**    **WO-A2-2007/067690**
**WO-A2-2010/018542**    **JP-A- 2014 100 249**
**US-A1- 2005 124 892**    **US-A1- 2011 295 579**

• **MISRA ET AL: "A study of solitary waves in a
tapered aorta by using the theory of solitons",
COMPUTERS & MATHEMATICS WITH
APPLICATIONS, ELSEVIER, AMSTERDAM, NL,
vol. 54, no. 2, 8 June 2007 (2007-06-08), pages
242-254, XP022109278, ISSN: 0898-1221, DOI:
10.1016/J.CAMWA.2006.12.025**
• **PANNIER, BM ET AL.: 'Methods and devices for
measuring arterial compliance in humans.',
[Online] 01 January 2002, pages 743 - 753,
XP055401311 Retrieved from the Internet:
<URL:HTTP://WWW.ARTERIOGRAPH.HU/DOWN
LOADS/A
STIFFNESS/METHODS-DEVICES-2002.PDF>**

EP 3 220 811 B1

**(Cont. next page)**

- O'ROURKE, M.: 'Arterial stiffness, systolic blood pressure, and logical treatment of arterial hypertension.', [Online] April 1990, pages 339 - 347, XP055187195 Retrieved from the Internet: <URL:HTTP://HYPER.AHAJOURNALS.ORG/CONTENT/1 5/4/339.FULL.PDF>
- TERRY, JD ET AL.: 'Peak systolic velocity and flow volume increase with blood pressure in low resistance systems.' JOURNAL OF ULTRASOUND IN MEDICINE. vol. 14, no. 3, 01 March 1995, pages 199 - 203, XP055444914 DOI: 10.7863/JUM.1995.14.3.199
- HOLZAPFEL, GA ET AL.: 'Constitutive modelling of arteries.' PROCEEDINGS OF THE ROYAL SOCIETY A: MATHEMATICAL, PHYSICAL AND ENGINEERING SCIENCES vol. 466, no. 2118, 01 January 2010 - 31 March 2010, pages 1551 - 1597, XP055441800 DOI: 10.1098/RSPA.2010.0058
- KONIG, G ET AL.: 'Mechanical properties of completely autologous human tissue engineered blood vessels compared to human saphenous vein and mammary artery.' BIOMATERIALS vol. 30, no. 8, 01 March 2009, pages 1542 - 1550, XP025876350 DOI: 10.1016/J.BIOMATERIALS.2008.11.011

**Description**

FIELD

The disclosure relates to methods for determining at least one of a blood pressure, an arterial compliance parameter or an arterial distensibility of a subject.

BACKGROUND

Exemplary blood vessel analysis apparatuses and methods are disclosed in document WO 2014/077233 A1. The pulse wave, generated by left ventricular ejection, propagates at a velocity that has been identified as an important marker of atherosclerosis and cardiovascular risk. Increased pulse wave velocity (PWV) indicates an increased risk of stroke and coronary heart disease. This velocity is considered a surrogate marker for arterial compliance, is highly reproducible, and is widely used to assess the elastic properties of the arterial tree. Research shows that measurement of pulse wave velocity as an indirect estimate of aortic compliance could allow for early identification of patients at risk for cardiovascular disease. The ability to identify these patients would lead to better risk stratification and earlier, more cost-effective preventative therapy. Several studies have shown the influence of blood pressure and left ventricular ejection time (LVET) on pulse wave velocity.

Over the past decades, there has been ongoing research for better theoretical prediction of PWV. The clinical relationship between PWV and arterial stiffness is often based on classic linear models or the combination of the linear models, and measured results with an incorporated correction factor. Whereas linear models predict PWV as a function of only geometric and physical properties of the fluid and the wall, there is strong empirical evidence that PWV is also correlated to pressure and ejection time.

There exist no models that accurately describe PWV and flow accounting for the nonlinearities in an arterial segment. A model that would enable solution of the inverse problem of determination of blood pressure and aortic compliance for a PWV measure has yet to be developed.

SUMMARY

In accordance with one aspect of the present invention, there is provided a method for determining at least one of a blood pressure, an arterial compliance parameter, or an arterial distensibility of a subject as defined in the appended claims.

This aspect of the present disclosure will become apparent upon a review of the following detailed description and the claims appended thereto.

BRIEF DESCRIPTION OF THE DRAWINGS

Figure 1 is a diagram on the longitudinal cross section of the arterial wall at diastolic (10) and systolic (11) pressure;
Figure 2 is a graph of the dependence of distensibilty on transmural pressure and a flow velocity;
Figure 3 is a flowchart depicting creation of a blood pressure and distensibility lookup table;
Figure 4 is a format of a lookup table for blood pressure and distensibility;
Figure 5 is a flowchart depicting a method for determining blood pressure of a subject based on a PWV measure using a lookup table;
Figure 6 is a flowchart depicting a method for determining arterial compliance of a subject based on a PWV measure using a lookup table;
Figure 7 is an illustration of pressure pulse measurement on two locations in the same artery for determination of a systolic and diastolic PWV; and
Figure 8 is a flowchart depicting a method for determining the material characteristics of an arterial segment.

DETAILED DESCRIPTION

A fully nonlinear model of pressure and flow propagation in arterial segments is disclosed that enables determination of blood pressure and arterial compliance based on measures of pulse wave velocity and flow velocity following a calibration. The approach allows for determination of systolic and diastolic blood pressure. The approach allows for determination of a systolic and diastolic aortic compliance.

A noninvasive method for monitoring the blood pressure and arterial compliance of a patient based on measurements of a flow velocity and a pulse wave velocity is described. An embodiment uses a photoplethysmograph and includes a method to monitor the dynamic behavior of the arterial blood flow, coupled with a hemodynamic mathematical model of the arterial blood flow motion in a fully nonlinear vessel.

A derived mathematical model creates the patient specific dependence of a blood pressure versus PWV and blood flow velocity, which allows continuous monitoring of arterial blood pressure. The calibrated mathematical model presents an arterial compliance and a distensibility as a clinical marker of arterial stiffness. The disclosure is applicable for fully nonlinear elastic vessels that are commonly found in the major arteries, as well as smaller vessels that operate closer to the linear elastic regime.

The disclosure includes a fully nonlinear basic model for blood pressure wave propagation in compliant arteries. A nonlinear traveling wave model was used to investigate mechanisms underlying the effects of pressure, ejection time, ejection volume, geometric, and physical properties on PWV. A patient calibration procedure was developed that involves measurement of blood pressure and arterial dimensions (internal and external radii).

An embodiment includes blood pressure prediction using the model, per patient calibration, and the measurement of flow velocity and pressure wave velocity. An embodiment includes arterial compliance determination using the model, per patient calibration, and the measurement of flow velocity and pressure wave velocity.

A basic mathematical fluid-structure interaction model for pulse wave velocity (PWV) propagation incorporates the dynamics of incompressible flow in a compliant vessel. This one dimensional model simulating blood flow in arteries effectively describes pulsatile flow in terms of averages across the section flow parameters. Although it is not able to provide the details of flow separation, recirculation, or shear stress analysis, it accurately represents the overall and average pulsatile flow characteristics, particularly PWV.

Conservation of mass and momentum results in the following system of one dimensional equations

$$\frac{\partial A}{\partial t} + \frac{\partial}{\partial z}(uA) = 0 \tag{1}$$

$$\frac{\partial u}{\partial t} + \frac{\partial}{\partial z}\left(\frac{u^2}{2} + \frac{p}{\rho}\right) = 0 \tag{2}$$

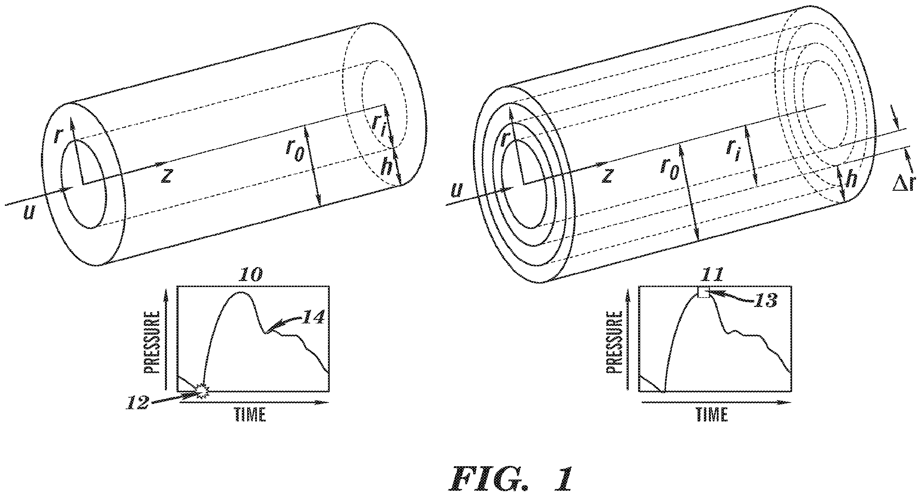

where $t$ is time, $z$ is the axial coordinate shown in Figure 1, $A = A(z,t)$ is the arterial cross-sectional area, $u = u(z,t)$ is the blood flow velocity, $\rho$ is blood density, $p = p(z,t)$ is blood pressure.

For an impermeable thin walled membrane, neglecting inertia forces, the vessel pressure - strain relationship is maintained by equilibrium condition as a function $p = p(\eta)$, based on relevant constitutive relations where $\eta$ is the circumferential strain (ratio of wall deflection to zero stress arterial radius (R)). Noting that $A = \pi R^2(1+\eta)^2$, and assuming that transmural pressure is a smooth function of a wall normal deflection (derivative $p_\eta = \partial p / \partial \eta$ exists at any point), the total system of equations can be presented in the following non-conservative form

$$\frac{\partial U}{\partial t} + H(U)\frac{\partial U}{\partial z} = 0 \tag{3}$$

where

$$U = \begin{bmatrix} \eta \\ u \end{bmatrix}; H = \begin{bmatrix} u & \dfrac{1+\eta}{2} \\ \dfrac{p_\eta}{\rho} & u \end{bmatrix} \tag{4}$$

We find the eigenvalues of $H(U)$ to be real and distinct. PWV is associated with the forward running wave velocity, i.e., the largest eigenvalue, hence it is identified as

$$PWV = u + \sqrt{\frac{1+\eta}{2\rho}p_\eta} \tag{5}$$

The partial derivative $p_\eta$ indicates sensitivity of pressure with respect to the wall normal deflection, and has a clear interpretation as tangent (incremental) moduli in finite strain deformation. In the general case, equation (5) is supplemented by appropriate constituent equations for a hyperelastic anisotropic arterial wall, accounting for finite deformation.

It is assumed that arterial wall is hyperelastic, incompressible, anisotropic, and undergoing finite deformation. After a few original loading cycles (preconditioning) the arterial behavior follows some repeatable, hysteresis free pattern with a typical exponential stiffening effect regarded as pseudo elastic. The strain energy density function *W* for the pseudo elastic constitutive relation may be presented in the form

$$W = \frac{1}{2}c(e^Q - 1) \tag{6}$$

where c is a material coefficient, and *Q* is the quadratic function of the Green-Lagrange strain components. For the finite inflation and extension of a thin walled cylindrical artery the following strain energy function is used

$$Q = a_{11}E_\theta^2 + 2a_{12}E_\theta E_z + a_{22}E_z^2 \tag{7}$$

where *c*, $a_{11}$, $a_{12}$, $a_{22}$ are material constants. The Cauchy stress components in circumferential and axial directions are:

$$\sigma_\theta = \lambda_\theta^2 \frac{\partial W}{\partial E_\theta} = c\lambda_\theta^2 e^Q s_\theta, \quad s_\theta = a_{11}E_\theta + a_{12}E_z$$

$$\sigma_z = \lambda_z^2 \frac{\partial W}{\partial E_z} = c\lambda_z^2 e^Q s_z, \quad s_z = a_{12}E_\theta + a_{22}E_z \tag{8}$$

With the geometry of the reference state determined, we define *R, Z, H* - as an internal radius, axial coordinate and a wall thickness in a stress free configuration, *r,z,h* - internal radius, axial coordinate and a wall thickness in a physiologically loaded configuration. The corresponding principal stretch ratios are

$$\lambda_\theta = r/R, \lambda_z = dz/dZ, \lambda_r = h/H \tag{9}$$

Assuming isochoric deformation incorporate the incompressibility condition as

$$\lambda_z \lambda_\theta \lambda_r = 1 \tag{10}$$

The Green-Lagrangian strain components relate to the principal stretch ratios of Eq. 12 by

$$E_i = \frac{1}{2}(\lambda_i^2 - 1), (i = \theta, z, r) \tag{11}$$

For the membrane thin walled cylindrical artery undergoing finite inflation and axial deformation, the load - stress relations follow from the static conditions

$$\sigma_\theta = \frac{pr}{h} = \frac{pR\lambda_\theta}{H\lambda_r} = \frac{pR}{H}\lambda_\theta^2\lambda_z \tag{12}$$

$$\sigma_z = \frac{F}{2\pi RH}\lambda_z = f\lambda_z$$

where F is the axial pretension force and *f* is the axial pre-stress per unit of cross section area of a load free vessel. A substitution back into equation (8) yields the desired relations:

$$\lambda_z^{-1}ce^Q s_\theta = \frac{pR}{H}$$
$$c\lambda_z e^Q s_z = f \tag{13}$$

The solution of equations (13), (11), (7) results in a load - strain relations, which with account of the identity $\lambda_\theta = \frac{r}{R} = \frac{r-R}{R} + 1 = \eta + 1$ converts into the *p* = *p*($\eta$) function, required by (5) to predict a wave front speed of propagation, i.e., PWV.

Arterial stiffness, or its reciprocals, arterial compliance and distensibility, may provide indication of vascular changes that predispose to the development of major vascular disease. In an isolated arterial segment filled with a moving fluid, compliance is defined as a change of a volume *V* for a given change of a pressure, and distensibilty as a compliance divided by initial volume. As functions of pressure the local (tangent) compliance *C* and distensibility *D* are defined as

$$C = \frac{dV}{dp}, D = \frac{C}{V} = \frac{dV}{Vdp} \tag{14}$$

Equations (14) determine arterial wall properties as local functions of transmural pressure.
We present equations (14) in the following equivalent form

$$C = \frac{dV/d\eta}{dp/d\eta} = \frac{2V}{p_\eta}, D = \frac{C}{V} = \frac{2}{p_\eta} \tag{15}$$

The classical results are generalized for the case of a hyperelastic arterial wall with account of finite deformation and flow velocity. To proceed, determine $p_\eta$ from Equation (5) and substitute in Equation (15), arriving at the following relations

$$D = \frac{1+\eta}{\rho(PWV-u)^2}, \quad C = VD \tag{16}$$

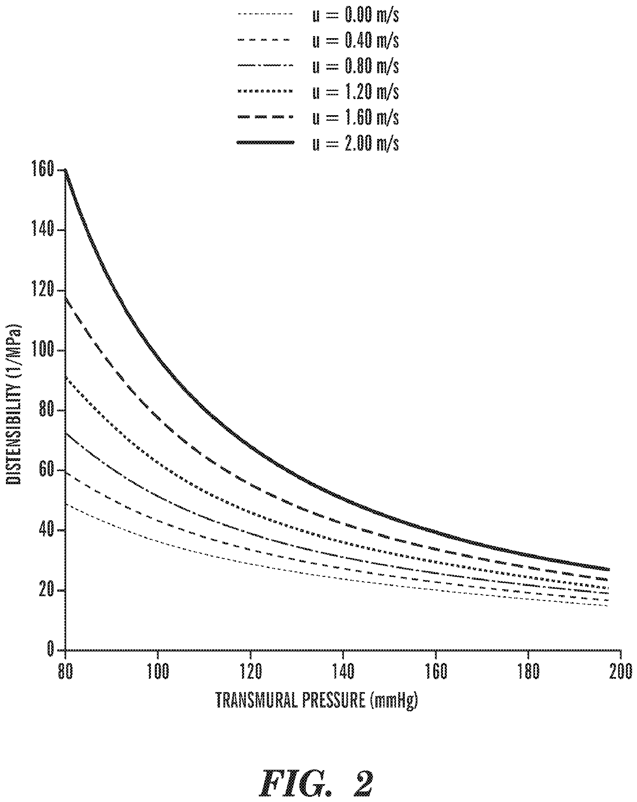

Figure 2 illustrates the dependency of distensibility on pressure and flow velocity. Since PWV is monotonically increasing with pressure, distensibility is a decreasing function. Unlike the classical Bramwell-Hill model, which being linked to the Moens-Korteweg wave speed predicts arterial distensibility as a constant irrespective to the pressure level, the present model predicts distensibility as a function of PWV, pressure and a blood flow.
Arterial constants can be defined based on the developed mathematical model. The Cauchy stress components based on Fung's energy are presented in equations (6), (7), and (8). Neglecting longitudinal stress ($\sigma_z$ = 0) in equation (8), we obtain

$$E_z = -\frac{a_{12}}{a_{22}}E_\theta \tag{17}$$

where the ratio $\frac{a_{12}}{a_{22}}$ is a counterpart of a Poisson coefficient in a linear isotropic elasticity.

It follows from Equations (7), (17) that a circumferential stress is a function of a circumferential strain and two material constants, *a* and c

$$\sigma_\theta = ac\lambda_\theta^2 e^{aE_\theta^2} E_\theta \qquad (18)$$

$$Q = aE_\theta^2 \qquad (19)$$

$$a = a_{11} - \frac{a_{12}^2}{a_{22}} \qquad (20)$$

The governing equation specifies an equilibrium condition

$$\sigma_\theta = ac\lambda_\theta^2 e^{aE_\theta^2} E_\theta = \frac{pr_i}{h}$$
$$h = r_o - r_i; \quad r_m = \frac{r_i + r_o}{2} \qquad (21)$$
$$\lambda_\theta = \frac{r_m}{R_m}; \quad E_\theta = \frac{\lambda_\theta^2 - 1}{2}$$

where $p$ is the transmural pressure, $r_i$ is the internal radius, $r_o$ is the outer radius, and $h$ is the wall thickness. Let us define $r_m$ as the mid radius of a loaded vessel, and $R_m$ as the mid radius for a stress free vessel. Measuring $r_i$ and $r_o$ corresponding to the pressure p leaves us with three unknowns ($a, c, R_m$) which need to be determined as a part of a calibration procedure. The calibration provides a calibrated model which can be individualized for each subject.

An embodiment of the calibration includes the use of published values for a population or segment of a population. Referenced values for material constants c, $a_{11}$, $a_{12}$, $a_{22}$ can be used to calculate the material constant *a* based on equation (20). The material constant *c* is used directly. A reference value for the mid radius in the stress free state ($R_m$) is used along with a reference value for the aterial wall thickness ($h$) and associated mid radius ($r_m$) for the loaded wall. The material parameters ($a, c, R_m$) along with the product of wall thickness and mid radius for the loaded wall ($hr_m$) can then be used to determine at least one of blood pressure and arterial compliance, e.g., distensibility, of a subject by measuring PWV and flow velocity.

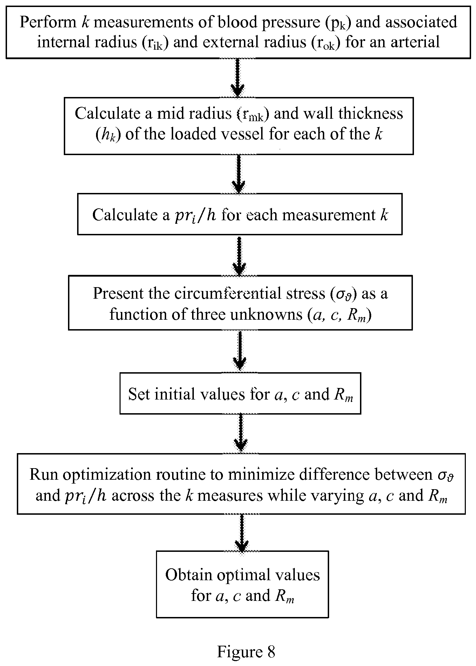

Another embodiment of the calibration is disclosed as follows and described in Figure 8. Assuming we have *k* measurements of the radius and pressure during a cardiac cycle or cycles, the following four variables are defined ($r_{ik}$, $r_{ok}$, $r_{mk}$, $p_k$). By using these sampled variables circumferential stress can be presented as a function of three unknowns

$$\sigma_\theta = \sigma_\theta(a, c, R_m) \qquad (22)$$

Now using a mathematical optimization, e.g., a least square (LS) minimization technique identifies ($a, c, R_m$)

$$LS = \sum_k [\sigma_\theta(a, c, R_m) - \frac{p_k r_{i_k}}{h_k}]^2 \xrightarrow[min]{} (a, c, R_m) \qquad (23)$$

The following nonlinear calibration method describes one approach that may be completed to determine arterial constants.

Step 1: Obtain *k* measurements, $k \geq 3$, for blood pressure - $p_k$, internal radius - $r_{ik}$; outer radius - $r_{ok}$, calculate mean radii $r_{mk} = 0.5(r_{ik} + r_{ok})$ and wall thicknesses $h_k = r_{ok} - r_{ik}$. For example, tonometry could be used to measure a continuous blood pressure to create the array of blood pressures, and Doppler speckle ultrasound could be used to measure artery radii to create the corresponding array of $r_{ik}, r_{ok}$.

Step 2: Run a least square minimization as in equation (23) to identify the three constants (two material constants $a, c$ and the mean radius $R_m$, in a load free condition). Substituting $\sigma_\theta$ in equation (23) with equation (18) results in

$$LS = \sum_k [ac\lambda_{\theta k}^2 e^{aE_{\theta k}^2} E_{\theta k} - \frac{p_k r_{i_k}}{h_k}]^2 \xrightarrow[min]{} (a, c, R_m) \qquad (24)$$

where $\lambda_{\theta k} = \frac{r_{mk}}{R_m}; \quad E_{\theta k} = \frac{\lambda_{\theta k}^2 - 1}{2}$ . LS is a function of measured parameters $p_k, r_{ik}, r_{mk}, h_k$ and unknown properties $(a, c, R_m)$, determined from the minimization procedure.

Since we have 3 unknowns, at least 3 sets of pressure and associated outer and inner radii are required.

In an embodiment, a calibrated model can be used to determine at least one of blood pressure and arterial compliance, e.g., distensibility, of a subject by measuring PWV and flow velocity.

With the three material properties ($a, c, R_m$) and the constant product ($hr_m$) a blood pressure may be estimated based on the equilibrium equation (21) rearranged to

$$p = \frac{ac\lambda_\theta^2 e^{aE_\theta^2} E_\theta h}{r_i} \qquad (25)$$

where the stretch ratio ($\lambda_\theta$) can be defined in terms of $\eta$

$$\lambda_\theta = \frac{r_m}{R_m} = (\frac{r_m - R_m}{R_m} + 1) = \eta + 1 \qquad (26)$$

The circumferential strain ($E_\theta$) can be defined in terms of $\eta$

$$E_\theta = (\lambda_\theta^2 - 1)/2 = \eta(\eta + 2)/2 \qquad (27)$$

Wall thickness follows from incompressibility conditions

$$hr_m = h_k r_{mk} \qquad (28)$$

where from

$$h = \frac{h_k r_{mk}}{r_m} = \frac{h_k r_{mk}}{R_m \lambda_\theta} = \frac{h_k r_{mk}}{R_m (\eta + 1)} \qquad (29)$$

Internal radius

$$r_i = r_m - 0.5h = \lambda_\theta R_m - 0.5h = (\eta + 1)R_m - 0.5h \qquad (30)$$

These formulations provide a relationship between pressure (p), circumferential strain ($\eta$), two arterial material parameters ($a$ and $c$) and two arterial geometric parameters ($R_m$ and the constant product $h_k r_{mk}$).

Equation (5) for PWV in arterial tissues can be re-arranged

$$p_\eta(1 + \eta) = 2\rho * PWV_f^2 \qquad (31)$$

where a flow corrected PWV ($PWV_f$) has been introduced ($PWV_f = PWV-u$).

This relation can be used in combination with equation (16) to define distensibility D in terms of the $p_\eta$

$$D = {}^2\!/p_\eta \qquad (32)$$

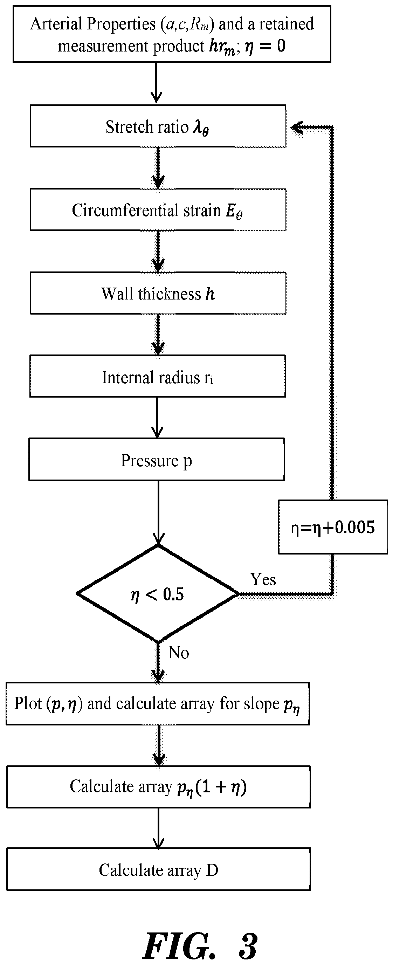

In an embodiment, a lookup table can be created for convenience to enable determination of a blood pressure and distensibility based on the 4 arterial parameters (can be subject specific) and measurement of PWV and flow velocity. A blood density $\rho$ is either measured or a value is assumed based on age and gender. As illustrated in an embodiment shown in Figure 3, the steps to create the blood pressure and distensibility lookup table are as follows:
Set $\eta=0$.

Using equation (26) calculate circumferential stretch ratio $\lambda_\theta$.

Using equation (27) calculate circumferential strain $E_\theta$.

Using equation (29) and any $h_k r_{mk}$ product from the calibration, calculate wall thickness A.

Using equation (30) calculate the internal radius $r_i$.

Using equation (25) calculate pressure $p$.

While $\eta<0.5$, $\eta= \eta+0.005$, go back to calculation of the circumferential stretch ratio or else continue.

Calculate the array for $p_\eta$ using the slope of the ($p,\eta$) curve.

Using equation (31) and the array of values for $\eta$ and $p_\eta$ calculate a 1D array for $p_\eta(1 + \eta)$.

Using equation (31) and the array of $p_\eta$, calculate D for each value of $\eta$.

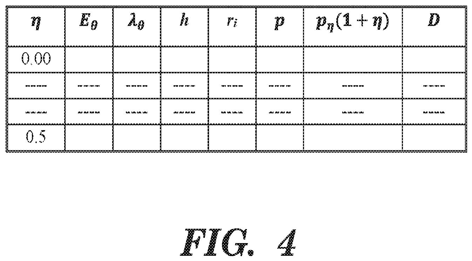

An example resultant array is shown in Figure 4. This lookup table enables determination of a subject blood pressure and distensibility by measuring $PWV_f$ and identifying the row (either directly or by interpolation) where

$$p_\eta(1 + \eta) = 2\rho * PWV_f^2$$

. This row also contains other relevant arterial parameters such as $E_\theta$, $\lambda_\theta$, $h$, and $r_i$ which can be extracted for that individual based on the measured $PWV_f$. Note that for determination of a systolic blood pressure and distensibility, a systolic PWV and a maximum flow velocity is used to calculate $PWV_f$. For determination of a diastolic blood pressure and distensibility, a diastolic PWV and a minimum (or zero) flow velocity is used to calculate $PWV_f$. Intermediate values of blood pressure and distensibility may also be determined based on the associated intermediate values of $PWV_f$ and flow velocity.

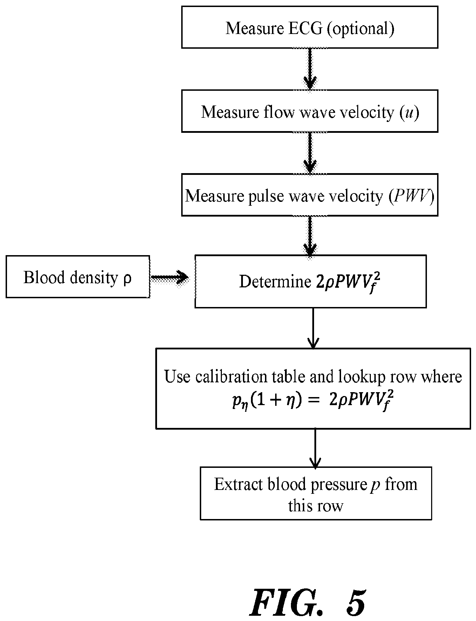

A method for monitoring a blood pressure of a subject is disclosed. The model is calibrated for the subject (or population) by a non-linear calibration. For example, a non-linear calibration as illustrated in Figure 3 can be performed. Once the calibration is complete, a patient specific lookup table can be formed for convenience as described in Figure 3 and as shown in Figure 4. The method for monitoring a subject's real time blood pressure is shown in Figure 5. An ECG measurement can be made for use as a timing reference for start of the pulse wave transit time, or to be used as a reference for determining acceptance time windows for other waveform features, or for averaging waveforms. An arterial flow velocity is measured providing minimum and maximum flow velocities, although a minimum could be assumed to be zero. An average flow velocity can also be measured. The flow velocity can also be estimated based on other measures or as a percentage of PWV. The pulse wave velocity is measured, ideally providing both a systolic and diastolic PWV. The subject (or population based) blood density $\rho$ are then used with the lookup table of Figure 4 to estimate blood pressure. The arrays of associated blood pressure ($p$), $PWV_f$ and distensibility ($D$) can also be used directly or with interpolation to estimate $p$ and $D$ based on the $PWV_f$ measure. The systolic PWV and the peak flow velocity are used to estimate a systolic blood pressure, while a diastolic PWV and the minimum flow velocity are used to estimate a diastolic blood pressure. Although other estimates and combinations may be used to estimate an average or systolic or diastolic blood pressure. PWV can be measured at the foot and the systolic flow velocity ($u$) can be added to it as an estimate of systolic $PWV_f$. Diastolic pressure could use the raw PWV measured at the foot and use either the minimum flow velocity or assume $u=0$. Other empirical or relational estimates for the systolic and diastolic $PWV_f$ can also be used.

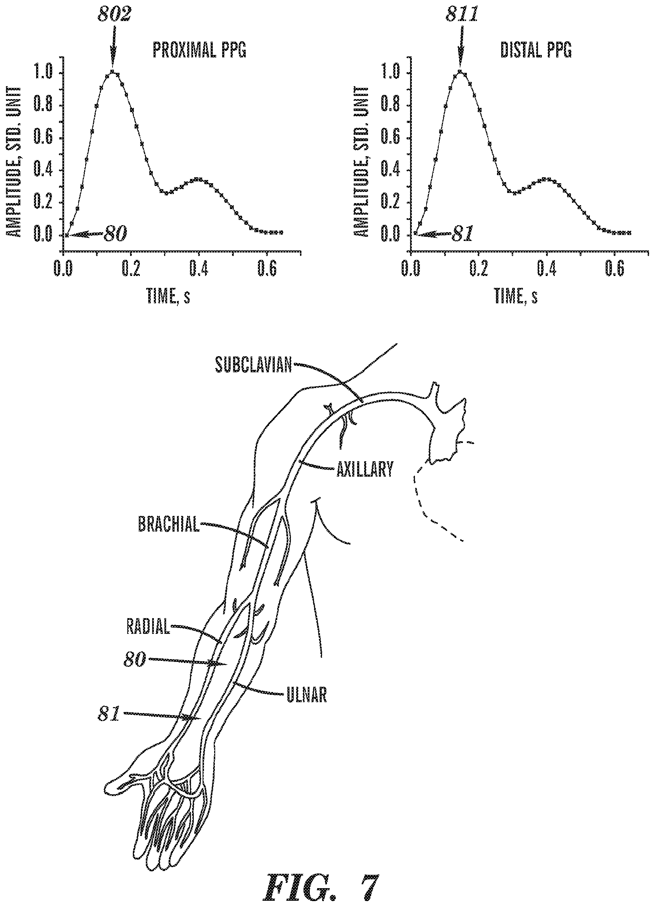

Determination of PWV includes measurement of the transit time of the pulse wave between two points, and a measure or estimate of the distance traveled. The PWV is the distance travelled divided by the time difference. This can be done by extracting the foot (Figure 1, item 12) or peak (Figure 1, item 13) of the pressure wave in two locations (proximal, distal) of the same artery, calculating the time difference between these two extracted features, and measuring the distance between these two measurement points. In one embodiment, this is done in the radial artery as shown in Figure 7 with measurements items 80 (proximal) and 81 (distal). Use of the foot location on the pressure or PPG waveform (items 801 and 810) will correlate to a diastolic PWV, while use of the peak location (items 802 and 811) will correlate to a systolic PWV. Two different arteries can also be used with a measurement or estimate of the arterial path distance between the two measurement points. The PWV can also be measured using the heart as the proximal measurement point with an electrocardiogram feature (e.g., the ECG r-wave) as the first time point, or by sensing when the aortic valve opens using a feature on measured waveforms or images such as the ballistocardiogram (BCG), ultrasound imaging, Doppler ultrasound, impedance plethysmography (IPG), or photoplethysmography (PPG) on the chest over the aorta. The distal pressure wave is then used as above to extract a second time point. The arterial distance between the aortic root and the distal measurement point is used in the calculation of PWV. This can be measured or estimated, and may be based on subject characteristics such as height, weight age and gender. The distal pressure wave can be measured using sensors such as tonometry, an arterial cuff, ultrasound, RF based arterial wall tracking, and PPG.

In some embodiments, the wave will propagate across multiple arterial segments between the proximal and distal points of pressure measurement. This measurement can be used in at least two ways. In the first form, average properties of the vessel segments, radius, and modulus will be considered so that the result corresponds to bulk average of the segment. In the second, the properties of individual arterial segments are determined. First, use the model to determine the relative transit time through each sequential arterial segment based on geometrical properties of each segment and assuming a similar pressure within all segments. Then using a solution method, such as minimization of a least squares or another method, solve for the PWV within each segment by recognizing that the total transit time (measured) is the sum of the transit time through each segment.

Measurement of flow velocity can be done using Doppler ultrasound, an inductive coil, MRI or CT scan with contrast agents. The flow velocity can be captured as a continuous wave, as a peak value, or a minimum value (including $u=0$). It is also possible to estimate flow velocity using related measures or with a scale factor. For example PWV can be measured using previously described techniques and flow velocity is then estimated as a percentage of PWV (for example $u = 0.2PWV$). Aortic flow velocity can be estimated through left ventricular ejection time (LVET), ejection volume (EV), and aortic cross-sectional area (CA) where $u = EV / (LVET * CA)$. Left ventricular ejection time ($LVET$) can be measured or estimated using a number of sensors (e.g. PPG, heart sound). Using PPG for example, the measure of LVET is the length of time from the foot of the PPG wave (Figure 1 item 12) to the dicrotic notch (item 14). Using heart sound, LVET is the time between the first and second heart sound. Ejection volume can be based on direct measurement for the subject (e.g. ultrasound, thermal dilution, etc) at rest and at exercise with subsequent scaling based on heart rate. The cross-sectional area can be directly measured by ultrasound imaging, MRI, or CT scans. EV and CA can also be based on subject specific parameters such as age, gender, height and weight. EV can also be measured or estimated using features from the BCG such as the amplitude of the j-wave or m-wave. An estimate of flow velocity in the periphery can be made based on scaling of the blood volume in that arterial tree branch, and relative arterial size as compared to the aorta. Although a direct measurement of flow velocity or an estimate based on PWV is preferred in the periphery.

Pressure can be measured using any approved technique, for example, brachial cuff, tonometry, or intra-arterial catheter. Ideally a continuous method (e.g., tonometry, intra-arterial) is used with a method of time synchronization to the flow and PWV measures (e.g. via ECG). However serial measures can also be used. Here the pressure $p$ can be systolic, diastolic, or any intermediate pressure (e.g., mean pressure) when coupled with the appropriate flow velocity (u). For example, systolic pressure could be associated with the peak flow velocity, and diastolic pressure could be associated with the lowest (or zero) flow velocity, or an average pressure could be associated with an average flow velocity.

An optional ECG can be measured across the chest or wrists. Other locations are also possible such as ear lobes, behind the ears, buttocks, thighs, fingers, feet or toes. PPG can be measured at the chest or wrist. Other locations such as the ear lobes, fingers, forehead, buttocks, thighs, and toes also work. Video analysis methods examining changes in skin color can also be used to obtain a PPG waveform. Flow velocity can be measured at the chest or wrist. Other locations for flow velocity measure are also possible such as the neck, arm and legs.

In one embodiment, the pulse transit time is measured based on aortic valve opening determined by the J-wave of the BCG waveform, and a PPG foot measured (e.g., Figure 1, item 12) from the thigh. The ECG r-wave may be used as a reference for determining acceptance windows for BCG and PPG feature delineations, or as a starting point for a PWV measure. The aorta distance is estimated from aortic root along the path of the aorta to the femoral artery at the thigh PPG measurement location. The PWV is calculated by dividing the aorta distance by the measured time difference (BCG J-wave to PPG foot). The minimum and maximum flow velocity is measured by ultrasound Doppler at the aortic root. A blood density $\rho$ is assumed based on age and gender of the subject. Using the measured $PWV_f$ as shown in Figure 5 the lookup table of Figure 4 is used to determine the associated pressure. To calculate $p_d$ the minimum measured flow velocity can be used in combination with an estimate of diastolic PWV, where diastolic PWV is calculated based on the BCG J-wave to PPG foot time difference. To calculate $p_s$ the peak flow velocity or a percentage of the peak flow velocity can be used in combination with an estimate of systolic PWV, where the systolic PWV is calculated based on the peak flow velocity time point to the PPG peak time difference. Under conditions where flow velocity is not measured, diastolic flow velocity can be assumed to be 0, while systolic flow velocity can be estimated as a percentage of PWV (e.g ~20%).

In another embodiment, the pulse transit time is measured from the carotid artery using tonometry, to the pressure pulse measured at thigh with a thigh cuff. The arterial distance is estimated from aortic root along the path of the aorta to the femoral artery at the thigh cuff measurement location. The PWV is calculated by dividing the arterial distance by the measured time difference. The foot to foot timing on the measured pressure pulses (e.g., Figure 7 items 801 and 810) is used to determine a diastolic pulse transit time and to calculate a diastolic PWV. The peak to peak timing (e.g., Figure 7, items 802 and 811) is used to determine a systolic pulse transit time and to calculate a systolic PWV. The peak flow velocity is estimated at 20% of the systolic PWV while the minimum flow velocity is estimated at zero. A blood density $\rho$ is assumed based on age and gender of the subject. Using the measured $PWV_f$, as shown in Figure 5, the lookup table of Figure 4 is used to determine the associated pressure. To calculate $p_d$ the minimum measured flow velocity can be used in combination with the diastolic PWV. To calculate $p_s$ the peak flow velocity is used in combination with the systolic PWV.

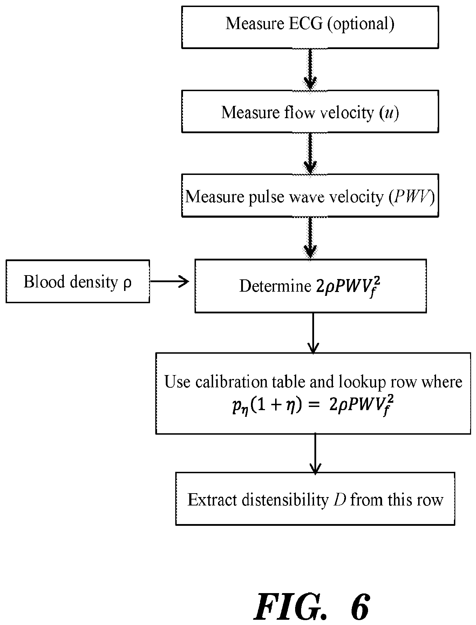

A method for monitoring an arterial compliance of a subject is disclosed. The model is calibrated for the subject (or population) by a non-linear calibration. For example, a non-linear calibration as illustrated in Figure 8 can be performed. Once the calibration is complete, a patient specific lookup table can be formed for convenience described in Figure 3 and as shown in Figure 4. The method for monitoring a subject's arterial compliance is shown in Figure 6. An optional ECG measurement can be made for use as a timing reference for start of the pulse wave transit time, or to be used as a reference for determining acceptance time windows for other waveform features, or for averaging waveforms. An arterial flow velocity is measured providing minimum and maximum flow velocities, although a minimum could be assumed to be zero. An average flow velocity can also be measured. The flow velocity can also be estimated based on other measures or as a percentage of PWV. The pulse wave velocity is measured, ideally providing both a systolic and diastolic PWV. The subject (or population based) blood density $\rho$ is then used with the patient specific lookup Figure 4, to determine compliance as shown in Figure 6 and equation 16.

The systolic PWV and the peak flow velocity are used to determine a systolic distensibility, while a diastolic PWV and the minimum flow velocity are used to estimate a diastolic distensibility. Although other estimates and combinations may be used to determine the subject distensibility parameter.

In one embodiment, the pulse transit time is measured based on aortic valve opening determined by the J-wave of the BCG waveform, and a PPG foot measured (e.g., Figure 1, item 12) from the thigh. The aorta distance is estimated from aortic root to femoral artery at the thigh PPG measurement location. The PWV is calculated by dividing the aorta distance by the measured time difference (BCG J-wave to PPG foot). The minimum flow velocity is assumed to be zero, enabling distensibility calculation without a direct flow measurement. The flow corrected PWV is calculated (in this embodiment $PWV_f$=PWV since $u$=0). A blood density $\rho$ is assumed based on age and gender of the subject with the patient specific lookup Figure 4 to determine compliance as shown in Figure 6 and equation 16. Measures of a systolic PWV, peak flow velocity, may also be used to determine D.

In another embodiment, the pulse transit time is measured from the carotid artery using tonometry, to the pressure pulse measured at thigh with a thigh cuff. The arterial distance is estimated from aortic root along the path of the aorta to the femoral artery at the thigh cuff measurement location. The PWV is calculated by dividing the arterial distance by the measured time difference. The foot to foot timing on the measured pressure pulses (e.g., Figure 8 items 801 and 810) are used to determine a diastolic pulse transit time and to calculate a diastolic PWV. The peak to peak timing (e.g., Figure 7, items 802 and 811) is used to determine a systolic pulse transit time and to calculate a systolic PWV. The peak flow velocity is estimated at 20% of the systolic PWV while the minimum flow velocity is estimated at zero. A blood density $\rho$ is assumed based on age and gender of the subject with the patient specific lookup Figure 4 to determine compliance as shown in Figure 6 and equation 16.

The disclosure will be further illustrated with reference to the following specific examples. It is understood that these examples are given by way of illustration and are not meant to limit the disclosure or the claims to follow.

EXAMPLE

Paper Example, Blood Pressure and Distensibility

This paper example uses referenced values for an aorta $c$=120123Pa, $a_{11}$=0.320, $a_{12}$=0.068, $a_{22}$=0.451. The reduced constant 'a' is calculated based on equation 20 ($a$=0.31). In addition reference values were used for the mid radius for the stress free vessel $R_m$=0.009m, the aortic wall thickness h=0.00211m, and the mid radius of the loaded wall $r_m$ = 0.011. A computer routine executed the functions outlined in Figure 3 to calculate an array of $p_\eta(1 + \eta)$, D, and the associated pressures ($p$) for each value of $\eta$. Equation (31) was then used along with a reference value of blood density $\rho$=1060kg/m$^3$ to create an array of associated $PWV_f$ for each value of $\eta$. The pulse transit time is measured based on time synchronized tonometry at the carotid artery and at the femoral artery, each providing a pressure pulse waveform. The equivalent aortic distance between the two measurement points is measured at for example 1.0m. The foot to foot time difference between the two tonometry waveforms is measured at for example 200 $ms$. The diastolic PWV is then calculated as 1.0m/200 $ms$ = 5.0$m/s$. From the same tonometry waveforms the peak to peak time difference is measured at for example 140 *ms.* The systolic PWV is then calculated as 1.00*m*/140 *ms* = 7.14*m/s*. Flow velocity is measured using Doppler ultrasound at the aortic root, with the minimum taken as the diastolic flow velocity $u_d = 0.00\,{}^m\!/_s$ , and the maximum taken as the systolic flow velocity $u_s = 1.10\,{}^m\!/_s$ . A flow corrected PWV is calculate at systolic blood pressure (PWV$_f$ = 7.14m/s - 1.10m/s = 6.04m/s) with the associated systolic blood pressure determined from the array as $p_s$=138 mmHg. A flow corrected PWV is calculate at diastolic blood pressure (PWV$_f$ = 5.0m/s -0.0m/s = 5.0m/s) with the associated diastolic blood pressure determined from the array as $p_d$=84 mmHg. The arterial compliance parameter distensibility (D) is determined from the systolic and diastolic *PWV$_f$* measures and the associated array elements. In this paper example the diastolic distensibility is identified as D = $57.6\,\dfrac{1}{MPa}$ , and the systolic distensibility is identified as $D=31.3\,\dfrac{1}{MPa}$.

**Claims**

1. A method for determining at least one of a blood pressure, an arterial compliance parameter, or an arterial distensibility of a subject, the method comprising;

   measuring a value for pulse wave velocity within an arterial segment or segments of a subject;
   providing a value for flow velocity within the arterial segment or segments of the subject;
   providing a value for blood density of the subject;
   determining values of material characteristics of an artery; and
   calculating, by a computer, at least one of a blood pressure, an arterial compliance parameter, or an arterial distensibility of the subject by inputting the value for pulse wave velocity, the value for flow velocity, the value for blood density, and the values of the material characteristics of the artery into a calibrated model of a fluid-structure interaction incorporating conservation of mass and momentum for a fluid, and non-linear elasticity of a structure.

2. The method of claim 1, wherein the value for flow velocity is measured directly from the subject.

3. The method of claim 1, wherein the value for flow velocity is estimated on the value for pulse wave velocity for the subject.

4. The method of claim 1, wherein the blood pressure is a systolic blood pressure.

5. The method of claim 1, wherein the blood pressure is a diastolic blood pressure.

6. The method of claim 4, wherein a peak pulse wave velocity is associated with the systolic blood pressure.

7. The method of claim 5, wherein a minimum pulse wave velocity is associated with the diastolic blood pressure.

8. The method of claim 4, wherein a peak flow velocity is associated with the systolic blood pressure.

9. The method of claim 5, wherein a minimum flow velocity is associated with the diastolic blood pressure.

10. The method of claim 1, wherein the value for pulse wave velocity is measured using at least one of photoplethysmography, impedance plethysmography, doppler ultrasound, an electrocardiogram, or a ballistocardiogram.

11. The method of claim 1, wherein determining the values of the material characteristics of an artery includes calculating at least one material constant using reference values associated with a segment of a population.

12. The method of claim 1, wherein the values of the material characteristics of an artery include *a* and *c*, $R_m$, where *a* is a reduced material constant, $a = a_{aa} - \dfrac{a_{12}^2}{a_{22}}$, $a_{11}$; $a_{12}$, $a_{22}$ are constants characterizing anisotropy of an arterial wall, c is a material constant, and $R_m$ is a mean wall radius in a load free state.

13. The method of claim 1, wherein the values of the material characteristics of an artery include one or more material constants associated with a strain energy density function representative of a pseudo-elastic behavior of the artery.

**Patentansprüche**

1. Verfahren zum Bestimmen von zumindest einem von einem Blutdruck, einem arteriellen Compliance-Parameter oder einer arterieller Dehnbarkeit eines Subjekts, das Verfahren umfassend;

   Messen eines Wertes für die Pulswellengeschwindigkeit innerhalb eines Arteriensegments oder von Arteriensegmenten eines Subjekts;
   Bereitstellen eines Wertes für die Strömungsgeschwindigkeit innerhalb des Arteriensegments oder der Arteriensegmente des Subjekts;
   Bereitstellen eines Wertes für die Blutdichte des Subjekts;
   Bestimmen von Werten der Materialcharakteristika einer Arterie; und
   Berechnen von zumindest einem von einem Blutdruck, einem arteriellen Compliance-Parameter oder einer arteriellen Dehnbarkeit des Subjekts durch einen Computer durch Eingeben des Wertes für die Pulswellengeschwindigkeit, des Wertes für die Strömungsgeschwindigkeit, des Wertes für die Blutdichte und der Werte der Materialcharakteristika der Arterie in ein kalibriertes Modell einer Fluid-Struktur-Wechselwirkung, das die Erhaltung von Masse und Impuls für ein Fluid und die nichtlineare Elastizität einer Struktur berücksichtigt.

2. Verfahren nach Anspruch 1, wobei der Wert für die Strömungsgeschwindigkeit direkt am Subjekt gemessen wird.

3. Verfahren nach Anspruch 1, wobei der Wert für die Strömungsgeschwindigkeit anhand des Wertes für die Pulswellengeschwindigkeit für das Subjekt geschätzt wird.

4. Verfahren nach Anspruch 1, wobei der Blutdruck ein systolischer Blutdruck ist.

5. Verfahren nach Anspruch 1, wobei der Blutdruck ein diastolischer Blutdruck ist.

6. Verfahren nach Anspruch 4, wobei dem systolischen Blutdruck eine Spitzen-Pulswellengeschwindigkeit zugeordnet ist.

7. Verfahren nach Anspruch 5, wobei dem diastolischen Blutdruck eine Minimum-Pulswellengeschwindigkeit zugeordnet ist.

8. Verfahren nach Anspruch 4, wobei dem systolischen Blutdruck eine Spitzen-Strömungsgeschwindigkeit zugeordnet ist.

9. Verfahren nach Anspruch 5, wobei dem diastolischen Blutdruck eine Minimum-Strömungsgeschwindigkeit zugeordnet ist.

10. Verfahren nach Anspruch 1, wobei der Wert für die Pulswellengeschwindigkeit unter Verwendung von zumindest einer/m von Photoplethysmographie, Impedanzplethysmographie, Doppler-Ultraschall, einem Elektrokardiogramm oder einem Ballistokardiogramm gemessen wird.

11. Verfahren nach Anspruch 1, wobei das Bestimmen der Werte der Materialcharakteristika einer Arterie das Berechnen zumindest einer Materialkonstanten unter Verwendung von Referenzwerten einschließt, die einem Segment einer Population zugeordnet sind.

12. Verfahren nach Anspruch 1, wobei die Werte der Materialcharakteristika einer Arterie $\alpha$ und $c$, $R_m$, einschließen,

$$a = a_{aa} - \frac{a_{12}^2}{a_{22}}$$

wobei $a$ eine reduzierte Materialkonstante ist, $a_{11}$; $a_{12}$, $a_{22}$ Konstanten sind, die die Anisotropie einer Arterienwand charakterisieren, $c$ eine Materialkonstante ist und $R_m$ ein mittlerer Wandradius in einem belastungsfreien Zustand ist.

13. Verfahren nach Anspruch 1, wobei die Werte der Materialcharakteristika einer Arterie eine oder mehrere Materialkonstanten einschließen, die einer Dehnungsenergiedichtefunktion zugeordnet sind, die repräsentativ für ein pseudoelastisches Verhalten der Arterie ist.

## Revendications

1.  Méthode permettant de déterminer au moins un élément parmi une tension artérielle, un paramètre de compliance artérielle ou une capacité de dilatation artérielle d'un sujet, la méthode comprenant ;

    la mesure d'une valeur de vitesse d'onde de pouls au sein d'un segment ou de segments artériels d'un sujet ;
    la fourniture d'une valeur pour la vitesse d'écoulement à l'intérieur du ou des segments artériels du sujet ;
    la fourniture d'une valeur pour la densité sanguine du sujet ;
    la détermination de valeurs de caractéristiques matérielles d'une artère ; et
    le calcul, au moyen d'un ordinateur, d'au moins un élément parmi une tension artérielle, un paramètre de compliance artérielle ou une capacité de dilatation artérielle du sujet en entrant la valeur de la vitesse de l'onde de pouls, la valeur de la vitesse d'écoulement, la valeur de la densité sanguine et les valeurs des caractéristiques matérielles de l'artère dans un modèle étalonné d'une interaction fluide-structure intégrant la conservation de la masse et de la quantité de mouvement pour un fluide et l'élasticité non linéaire d'une structure.

2.  Méthode selon la revendication 1, ladite valeur de la vitesse d'écoulement étant mesurée directement à partir du sujet.

3.  Méthode selon la revendication 1, ladite valeur de la vitesse d'écoulement étant estimée sur la valeur de la vitesse de l'onde de pouls pour le sujet.

4.  Méthode selon la revendication 1, ladite tension artérielle étant une tension artérielle systolique.

5.  Méthode selon la revendication 1, ladite tension artérielle étant une tension artérielle diastolique.

6.  Méthode selon la revendication 4, une vitesse maximale d'onde de pouls étant associée à la tension artérielle systolique.

7.  Méthode selon la revendication 5, une vitesse minimale d'onde de pouls étant associée à la tension artérielle diastolique.

8.  Méthode selon la revendication 4, une vitesse d'écoulement maximale étant associée à la tension artérielle systolique.

9.  Méthode selon la revendication 5, une vitesse d'écoulement minimale étant associée à la tension artérielle diastolique.

10. Méthode selon la revendication 1, ladite valeur de la vitesse d'onde de pouls étant mesurée à l'aide d'au moins une technique parmi la photopléthysmographie, la pléthysmographie à impédance, l'échographie Doppler, un électrocardiogramme ou un ballistocardiogramme.

11. Méthode selon la revendication 1, ladite détermination des valeurs des caractéristiques matérielles d'une artère comprenant le calcul d'au moins une constante matérielle à l'aide de valeurs de référence associées à un segment d'une population.

12. Méthode selon la revendication 1, lesdites valeurs des caractéristiques matérielles d'une artère comprenant $a$ et c,

$$a = a_{aa} - \frac{a_{12}^2}{a_{22}}$$

$R_m$, où $a$ est une constante matérielle réduite, , $a_{11}$ ; $a_{12}$, $a_{22}$ sont des constantes caractérisant l'anisotropie d'une paroi artérielle, c est une constante matérielle et $R_m$ est un rayon de paroi moyen dans un état exempt de charge.

**13.** Méthode selon la revendication 1, lesdites valeurs des caractéristiques matérielles d'une artère comprenant une ou plusieurs constantes matérielles associées à une fonction de densité d'énergie de déformation représentant un comportement pseudo-élastique de l'artère.

*FIG. 1*

*FIG. 2*

Arterial Properties $(a,c,R_m)$ and a retained measurement product $hr_m$; $\eta = 0$

↓

Stretch ratio $\lambda_\theta$

↓

Circumferential strain $E_\theta$

↓

Wall thickness $h$

↓

Internal radius $r_i$

↓

Pressure p

↓

$\eta < 0.5$ — Yes → $\eta = \eta + 0.005$ →

No

↓

Plot $(p, \eta)$ and calculate array for slope $p_\eta$

↓

Calculate array $p_\eta(1 + \eta)$

↓

Calculate array D

# FIG. 3

| $\eta$ | $E_\theta$ | $\lambda_\theta$ | $h$ | $r_i$ | $p$ | $p_\eta(1+\eta)$ | $D$ |
|---|---|---|---|---|---|---|---|
| 0.00 | | | | | | | |
| ---- | ---- | ---- | ---- | ---- | ---- | ---- | ---- |
| ---- | ---- | ---- | ---- | ---- | ---- | ---- | ---- |
| 0.5 | | | | | | | |

## FIG. 4

Measure ECG (optional)

Measure flow wave velocity ($u$)

Measure pulse wave velocity ($PWV$)

Blood density $\rho$ → Determine $2\rho PWV_f^2$

Use calibration table and lookup row where
$$p_\eta(1 + \eta) = 2\rho PWV_f^2$$

Extract blood pressure $p$ from this row

# FIG. 5

**FIG. 6**

FIG. 7

Perform $k$ measurements of blood pressure ($p_k$) and associated internal radius ($r_{ik}$) and external radius ($r_{ok}$) for an arterial

⬇

Calculate a mid radius ($r_{mk}$) and wall thickness ($h_k$) of the loaded vessel for each of the $k$

⬇

Calculate a $pr_i/h$ for each measurement $k$

⬇

Present the circumferential stress ($\sigma_\vartheta$) as a function of three unknowns ($a$, $c$, $R_m$)

⬇

Set initial values for $a$, $c$ and $R_m$

⬇

Run optimization routine to minimize difference between $\sigma_\vartheta$ and $pr_i/h$ across the $k$ measures while varying $a$, $c$ and $R_m$

⬇

Obtain optimal values for $a$, $c$ and $R_m$

Figure 8

**EP 3 220 811 B1**

**Patent documents cited in the description**

- WO 2014077233 A1